(19) Europäisches Patentamt
European Patent Office
Office européen des brevets

(11) Publication number: **0 029 202**
**B1**

(12) # EUROPEAN PATENT SPECIFICATION

(45) Date of publication of patent specification: **13.04.83**

(21) Application number: **80106952.7**

(22) Date of filing: **11.11.80**

(51) Int. Cl.$^3$: **C 07 D 501/36,**
C 07 D 501/06,
C 07 D 257/04

(54) **Novel tetrazole-5-thiol esters and process for preparing cefamandole using same.**

(30) Priority: **16.11.79 JP 148485/79**
**16.11.79 JP 148486/79**
**06.03.80 JP 27303/80**
**07.03.80 JP 28043/80**

(43) Date of publication of application:
**27.05.81 Bulletin 81/21**

(45) Publication of the grant of the patent:
**13.04.83 Bulletin 83/15**

(84) Designated Contracting States:
**AT BE CH DE FR GB IT LI LU NL SE**

(56) References cited:
**EP - A - 0 004 570**
**GB - A - 1 053 587**

(73) Proprietor: **Asahi Kasei Kogyo Kabushiki Kaisha**
**2-6, Dojimahama 1-chome Kita-ku**
**Osaka-shi Osaka 530 (JP)**

(72) Inventor: **Shibuya, Chisei**
**2-1 Kawanarijima**
**Fuji-shi Shizuoka-ken (JP)**
Inventor: **Murakami, Masahiro**
**Kita 9-Jyo 1988-4, Okitacho**
**Nobeoka-shi, Miyazaki (JP)**
Inventor: **Kobayashi, Masateru**
**Dai 2 Koanryo 6-3, Kitashinkoji**
**Nobeoka-shi Miyazaki (JP)**
Inventor: **Sone, Takanori**
**Room 131 Hamayama Apartment, 4-1**
**Midorigaoka 2-chome Nobeoka-shi Miyazaki (JP)**
Inventor: **Yamamoto, Kimiyo**
**1607, Shiohamacho 1-chome**
**Nobeoka-shi Miyazaki (JP)**

(74) Representative: **Boeters, Hans Dietrich, Dr. et al,**
**Boeters, Bauer & Partner Thomas-Wimmer-Ring 14**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Novel tetrazole-5-thiol esters and process for preparing cefamandole using same

GB 1 053 587 describes specific tetrazole-5-thiol esters.

This invention relates to noval tetrazole-5-thiol esters useful as an acylating agent for amines and hydrazines, particularly as active esters for preparing cephalosporin compounds and a process for the preparation of cefamandole using the thiol esters.

Cefamandole is well known as an antibacterial agent and is reported by many documents such as Antimicrobial Agents & Chemotherapy 1(3) 221—234 (1972) by Wick et al., U.S. Patent No. 3,641,021 Example 4 by Ryan, French Patent No. 7,310, 112 by Greene and U.S. Patent No. 3,796,801 Example 1 by Guarini. EP 79 100 700.8=4 570 describes a reaction of 1,3,4-thiadiazole-5-thiol ester with 7-aminocephalosporanic acid or its esters. However an application of this reaction type with the use of the said acid or its esters for the preparation of cefamandole is not satisfactory with respect to shortened reaction times or increased yields.

An object of this invention is to provide a novel tetrazole-5-thiol ester which can be used as a starting material for preparing cefamandole and to provide a method of the production thereof.

Another object of this invention is to provide a method of preparing cefamandole with a higher yield from the tetrazole-5-thiol ester.

Accordingly, the present invention in one embodiment provides a tetrazole-5-thiol ester of the formula:

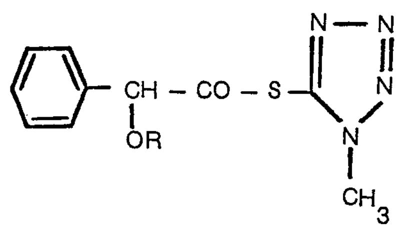

(I)

wherein R is a hydrogen atom or a formyl group.

The present invention in another embodiment provides a process for preparing the above described compound of formula (I).

In a further embodiment, the invention provides a process for preparing a compound of the formula:

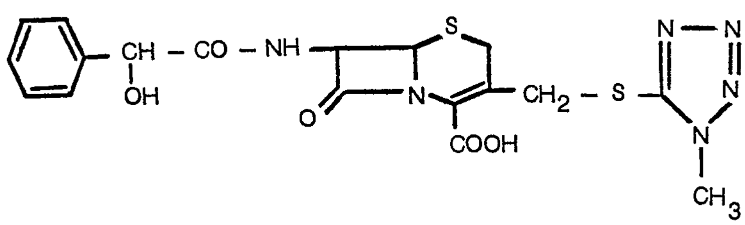

(II)

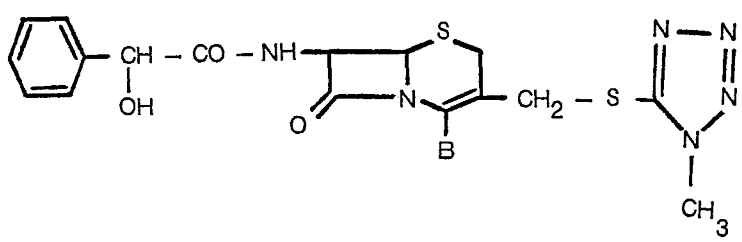

(IIa)

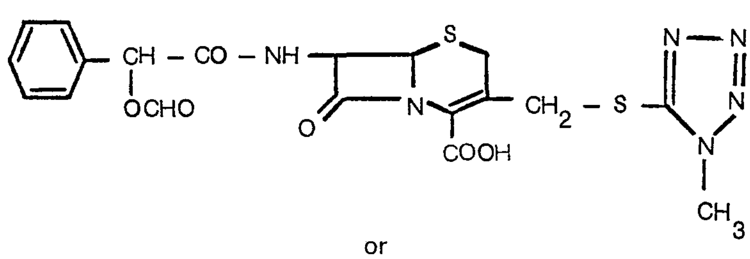

(IIb)

or

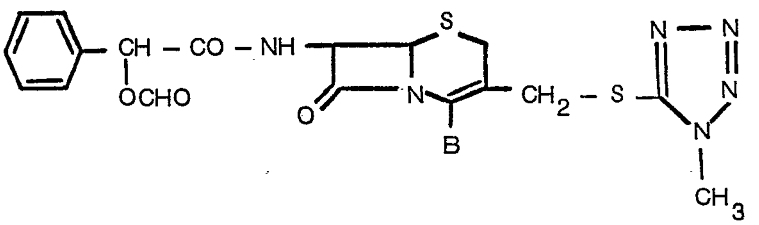

(IIc)

or salts of compounds of formulas (II) and (IIb), respectively, wherein B is a carboxylic acid ester group, which comprises reacting the tetrazole-5-thiol ester of the formula (I) with a compound of the formula (IIIa):

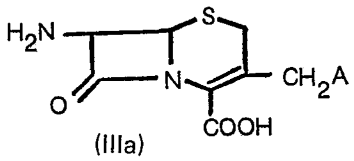

(IIIa)

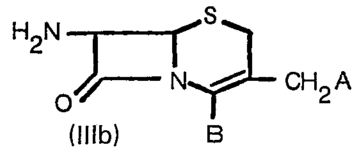

(IIIb)

or its salt or with a compound of the formula (IIIb) wherein A is a benzimidazol-2-ylthio group or a 1-methyl-1H-tetrazol-5-ylthio group, and B has the same meaning as above.

The tetrazole-5-thiol esters of the formula (I) of this invention are D-(—)-mandeloyl-1-methyl-1H-tetrazol-5-ylthiol ester and O-formyl-D-(—)-mandeloyl-1-methyl-1H-tetrazol-5-ylthiol ester.

These compounds of the formula (I) of this invention are novel compounds and useful as antibacterial agents.

Also, these compounds of the formula (I) of this invention are useful as acylating agents for amines and hydrazines, particularly as active esters for preparing cephalosporin compounds.

The tetrazole-5-thiol esters of the formula (I) of this invention can be prepared by reacting a 1-methyl-1H-tetrazole-5-thiol or its derivative with a free carboxylic acid of D-(—)-mandelic acid or O-formyl-D-(—)-mandelic acid or a reactive derivative of the carboxylic group in the D-(—)-mandelic acid or O-formyl-D-(—)-mandelic acid optionally in a solvent.

The derivatives of the 1-methyl-1H-tetrazole-5-thiol which can be employed in this invention include the salts of sodium and potassium; the reaction products of trimethylaluminum and triethylaluminum; the trimethylsilyl derivative and the triethylsilyl derivative; and the trifluoracetates.

When the free carboxylic acid of D-(—)-mandelic acid or O-formyl-D-(—)-mandelic acid is used in this invention, a dehydrating agent for condensation is added to the reaction system. The dehydrating agents for condensation which can be employed in this invention include the conventional agents used in this field such as N,N′-dicyclohexylcarbodiimide, N,N′-diisopropylcarbodiimide, carbonyldiimidazol and N-cyclohexyl-N′-morpholinoethylcarbodiimide. Generally, the amount of the dehydrating agent is 0.5 to 10 moles and preferably 1 to 5 moles per mole free carboxylic acid, amounts outside these ranges are possible depending on the system selected.

When the reactive derivative of the carboxylic group is used, it is unnecessary to employ the dehydrating agent for condensation.

The reactive derivatives of the carboxylic group in the D-(—)-mandelic acid or O-formyl-D-(—)-mandelic acid which can be employed in this invention include the acid halides, the esters, the mixed acid anhydrides, the acid amides, the acid azides and the nitriles.

Specific examples of suitable derivatives include the acid chloride; the mixed acid anhydrides of a dialkylphosphoric acid; the mixed acid anhydrides of a phenylphosphoric acid; the mixed acid anhydride of a diphenylphosphoric acid; the mixed acid anhydride of a dibenzylphosphoric acid; the mixed acid anhydride of halogenated phosphoric acids such as chlorophosphoric and bromophosphoric acid; the mixed acid anhydride of a dialkylphosphorous acid; the mixed acid anhydride of sulfurous acid; the mixed acid anhydride or thiosulfuric acid; the mixed acid anhydride of sulfuric acid; the mixed acid anhydride of an alkylcarbonic acid; the mixed acid anhydride of an aliphatic carboxylic acid such as pivalic acid, pentanoic acid, isopentanoic acid, 2-ethylbutanoic acid, trichloroacetic acid and trifluoroacetic acid; the mixed acid anhydride of an aromatic carboxylic acid such as benzoic acid; the symmetric acid anhydrides; the acid amides of imidazole, a 4-substituted imidazole, dimethylpyrazole, triazole, tetrazole, ammonia, methylamine and dimethylamine; the esters such as methyl ester, ethyl ester, cyanomethyl ester, methoxymethyl ester, vinyl ester, propargyl ester, p-nitrophenyl ester, 2,4-dinitrophenyl ester, trichlorophenyl ester, pentachlorophenyl ester, methanesulfonylphenyl ester, phenylazophenyl ester, phenyl ester, phenylthioester, p-nitrophenylthioester, 2,4-dinitrophenylthioester, p-cresylthioester, carboxymethylthioester, pyranyl ester, pyridyl ester, 8-quinolylthioester, the ester of N,N-dimethylhydroxylamine, 1-hydroxy-2-(1H)-pyridone, N-hydroxysuccinimide or N-hydroxyphthalimide.

The 1-methyl-1H-tetrazole-5-thiol or its derivative is dissolved or suspended in a solvent and the solution or the suspension is added with the carboxylic acid, the reactive derivative of the carboxylic

group or solution or suspension thereof. As a general rule the reaction is carried out for about 30 minutes to about 24 hours. Reaction times outside this range are possible depending on the system selected.

Any solvent which is inert to the reactants and can dissolve or suspend the reactants can be employed in this invention.

Specific examples of suitable solvents include dioxane, ethyl acetate, methylene chloride, chloroform, hexane and aqueous solutions of sodium hydroxide or potassium hydroxide.

The reaction temperature which can be employed in the preparation of the tetrazole-5-thiol ester of the formula (I) may be varied within a wide range of temperatures. In general, the reaction temperature is about −20°C to about 50°C, and preferably 0°C to about 50°C. Reaction temperatures outside this range are possible depending on the system selected.

In general 0.5 to 2.0 moles and preferably 0.9 to 1.2 moles of the thiol or its derivative are reacted with 1 mole mandelic acid or its derivative. Other ratios are possible depending on the system selected. After the reaction is completed, the desired compound of 1-methyl-1H-tetrazole-5-thiol ester or its derivative of the formula (I) can be recovered from the reaction mixture and purified by the conventional techniques. For example, the solvent is evaporated from the reaction mixture and the crude product thus obtained is washed, dried or recrystallized.

Since the 1-methyl-1H-tetrazole-5-thiol ester or its derivative of the formula (I) has a very active thiol ester group

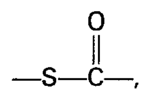

the compound can easily acylate amines, hydrazines, phenols or alcohols with a high yield.

The purification of this acylation product is very simple and this is a remarkable aspect of the invention. When the compound of the formula (I) of this invention is used as an acylating agent, 1-methyl-1H-tetrazole-5-thiol is precipitated with the proceeding of acylation. Accordingly, the product can be easily separated from the reaction mixture and purified. Further, as the 1-methyl-1H-tetrazole-5-thiol is amphoteric, it can be easily removed by washing with either aqueous solution of acid and alkali. Although the 1-methyl-1H-tetrazole-5-thiol might remain in the product, it can be easily removed by the above described washing. Accordingly, by using the compound of the formula (I) of this invention as an acylating agent, acylated compounds having high purity can be easily obtained.

The cefamandole having D-configuration in the side chain of the formula (II) of this invention can be prepared by reacting 1-methyl-1H-tetrazole-5-thiol ester or its derivative of the formula (I) with the compound of the formula:

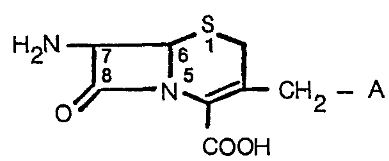

(IIIa)

wherein A is a benzimidazol-2-ylthio group or a 1-methyl-1H-tetrazol-5-ylthio group; or its salt; or its ester of the formula (IIIb):

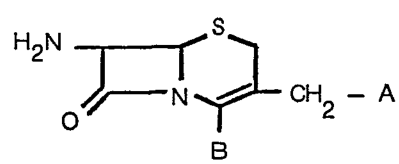

(IIIb)

wherein A is the same as defined above and B is a carboxylic acid ester group.

Suitable examples of the derivatives of the compound (IIIa) and (IIIb) which can be employed in this invention include the salts of alkali metals such as sodium and potassium; the salts of alkaline earth metals such as calcium and magnesium; the salts of nitrogen-containing organic bases such as trimethylamine, triethylamine, pyridine, N-methylpiperidine and N-methylmorpholine; and the esters (where the carboxylic acid group carries a usual protective group) which can be easily converted into the compound of the formula (IIIa) or its salt, for example, by catalytic reduction, chemical reduction, or hydrolysis, released by catalytic reduction, chemical reduction or under other mild conditions.

The preparation of the cefamandole of the formula (II) is generally conducted in a solvent. Any solvents which are inert to the reactants can be employed in this invention.

Specific examples of suitable solvents used include water, acetone, dioxane, acetonitrile, toluene, chloroform, methylene chloride, ethylene chloride, tetrahydrofuran, ethyl acetate, formic acid, pyridine, trifluoroacetic acid, N,N-dimethylformamide, methanol, ethanol, methoxy ethanol, diethyl ether, isopropyl ether, dimethyl sulfoxide and any mixtures of water with the above described organic solvents.

In general the temperature of the above described reaction which can be employed in this invention is about —50°C to about 100°C, and preferably about 0°C to about 80°C. The period of time of the above described reaction typically ranges from about 10 minutes to several tens of hours, and preferably from about 0.5 hour to about 6 hours. However, reaction temperatures and times outside the ranges given are possible depending on the system selected. The above described reaction is continued until the desired compound of the formula (II) is produced in a most appropriate amount.

Further, in the above described reaction an acid catalyst can be employed, if necessary. Specific examples of the acid catalysts which can be employed in this invention include inorganic acids such as hydrochloric acid and sulfuric acid; sulfonic acids such as paratoluenesulfonic acid and methanesulfonic acid; organic acids such as aliphatic carboxylic acids like propionic acid, and such as 1-methyl-1H-tetrazolyle-5-thiol. The inorganic acid and the thiol are preferred from the viewpoint of high yield and short reaction period.

The amount of the catalyst which can be employed is not specifically limited; however, generally, 0.01 to 0.1 mole per mole of the compound of the formula (IIIa) or (IIIb) are used and preferably 0.05 mole per mole of 7-amino-3-(benzimidazol-2-ylthiomethyl)-3-cephem-4-carboxylic acid.

In case of compounds of the formula (IIIa) or (IIIb) especially 7-amino-cephalosporanic acid or 7-amino-3-(1-methyl-1H-tetrazol-5-ylthiomethyl)-3-cephem-4-carboxylic acid, it is preferred that the reaction between the compound of the formula (I) and the compound of the formula (IIIa) or (IIIb) is conducted at a pH ranging from 1 to 10. A more preferred pH is 2 to 9.

Generally, the amount of the compound of the formula (I) which can be employed ranges from about 0.5 mole to about 2.0 moles and preferably from about 0.9 mole to about 1.2 moles per mole of the compound of the formula (IIIa) or (IIIb). However, other ratios are possible depending on the selected system.

The reaction products (II) or (IIb) or their salts are separated and collected by conventional methods from the reaction mixture.

The reaction products (IIa) or (IIc) are further subjected to a cleavage of the ester group, for example, by catalytic reduction, chemical reduction or hydrolysis in order to isolate compounds (II) and (IIb), respectively, or their pharmaceutically acceptable salts.

The O-formyl-cefamandole of the formula (IIb) thus obtained can be converted, by the conventional methods, into the salt of an alkali metal, the salt of ammonium and the salt of an alkaline earth metal.

Further, the O-formyl-cefamandole of the formula (IIb) or its salt can be converted into the cefamandole of the formula (II) by treating the O-formyl-cefamandole or its salt with an alkaline solution such as an aqueous solution of an alkali such as sodium carbonate and sodium bicarbonate.

The following examples are given to illustrate the present invention more specifically. However, it should be understood that the invention is in no way limited by these examples. Room and ambient temperature means about 25°C.

## Example 1

In 100 ml of anhydrous ethyl acetate were dissolved 2.32 g (0.02 mole) of 1-methyl-1H-tetrazole-5-thiol and to the solution were added 3.6 g (0.02 mole) or O-formyl-D-(—)-mandelic acid. Then to the solution was added 4.1 g (0.02 mole) of N,N'-dicyclohexylcarbodiimide with stirring at 5°C and the stirring was continued for 2 hours at 5°C. After the solution was left to stand overnight at 0°C, the precipitate thus formed was removed by filtration. The filtrate was washed twice with 90 ml of saturated aqueous sodium chloride solution in each time and dehydrated with magnesium sulfate anhydride, and then ethyl acetate was removed from the dehydrated product at room temperature under reduced pressure to give 5.2 g of an oily compound, i.e., O-formyl-D-(—)-mandelic acid 1-methyl-1H-tetrazol-5-ylthiol ester at a yield of 93%.

Infrared Absorption Spectrum:

nyc=0: 1740 $cm^{-1}$

Elemental Analysis Values:

| | | | | |
|---|---|---|---|---|
| Calculated (%): | C, 47.5; | H, 3.60; | N, 20.1; | S, 11.5 |
| Found (%): | C, 47.9; | H, 3.65; | N, 20.2; | S, 11.4 |

NMR Spectrum ($CDCl_3$), $\delta$ ppm:

3.86 (s, 3H), 6.40 (s, 1H), 7.45 (m, 5H), 8.27 (s, 1H)

Example 2

In a 1 l eggplant type flask having a tube packed with calcium chloride as a desiccant were charged 15.2 g of D-(—)-mandelic acid, 11.6 g of 1-methyl-1H-tetrazole-5-thiol and 500 ml of anhydrous ethyl acetate and stirred. The solution thus obtained was added with 20.6 g of N,N'-dicyclohexylcarbodiimide and stirred at 25°C for 14 hours. The reaction solution was filtered to remove the insoluble N,N'-dicyclohexylurea. The filtrate was distilled under reduced pressure. The oily compound thus obtained was dissolved in a small amount of ethyl acetate, and then was crystallized in a small amount of hexane. The crystals were filtered and dried by the conventional method to give 21.5 g of mandelic acid-1-methyl-tetrazol-5-ylthiol ester in the form of light brown crystals at a yield of 86%.

Elemental Analysis Values:

| | | | | |
|---|---|---|---|---|
| Calculated (%): | C, 48.0; | H, 4.0; | N, 22.4; | S, 12.8 |
| Found (%): | C, 47.8; | H, 4.3; | N, 22.5; | S, 12.6 |

NMR Spectrum ($CDCl_3$), $\delta$ ppm:
7.30 (m, 5H), 3.80 (s, 3H), 6.10 (s, 1H), 10.70 (s, 1H)

Mass Spectrum:
$M^+$ 250

Comparative Example 1

To an aqueous solution consisting of 0.84 g of sodium hydrogencarbonate, 1.36 g (0.005 mole) of 7-amino-cephalosporanic acid and 100 ml of water were added at once the solution containing 1.8 g (0.0065 mole) of O-formyl-D-(—)-mandelic acid-1-methyl-1H-tetrazol-5-ylthiol ester obtained in the above described Example 1 and 10 ml of acetone at room temperature. Then the solution was stirred at 50°C to 55°C for 3 hours. The reaction solution was washed with ether and was added with 3N hydrochloric acid to adjust the pH of the solution to 3, and then the solution was extracted twice with 100 ml of ethyl acetate in each time. The layer of ethyl acetate was washed with saturated aqueous sodium chloride solution. After the layer was dehydrated with anhydrous magnesium sulfate the ethyl acetate was evaporated and the residue dried. The residue thus obtained was pulverized by adding ether thereto to give powdery solid. The solid was filtered and was washed with ether and then dried to give 2.0 g of 7-(D-2-formyloxy-2-phenylacetoamide)-3-(1-methyl-1H-tetrazol-5-ylthiomethyl)-3-cephem-4-carboxylic acid (O-formylcefamandole) at a yield of 81%.

Elemental Analysis Values:

| | | | | |
|---|---|---|---|---|
| Calculated (%): | C, 46.5; | H, 3.67; | N, 17.1; | S, 13.1 |
| Found (%): | C, 46.7; | H, 3.75; | N, 17.0; | S, 13.0 |

NMR Spectrum (DMSO-$d_6$), $\delta$ ppm:
3.52 (s, 2H), 3.88 (s, 3H), 4.10 (s, 2H), 4.92 (d, 1H), 5.62 (q, 1H), 6.06 (s, 1H), 7.26 (s, 1H), 8.28 (s, 1H)

Example 3

The solution consisting of 1.8 g (0.005 mole) of 7-amino-3-(benzimidazol-2-ylthiomethyl)-3-cephem-4-carboxylic acid, 50 ml of N,N-dimethylformamide and 50 ml of water was heated at 60°C and to the solution was added 1.8 g (0.0065 mole) of O-formyl-D-(—)-mandelic acid-1-methyl-1H-tetrazol-5-ylthiol ester. Further, the solution was added with 5 ml of 0.5 N hydrochloric acid and stirred at 60°C for 3 hours. The reaction solution was washed with ether and then added with 1N hydrochloric acid to adjust the pH of the solution to 1.5. The obtained solution was extracted three times with 100 ml of ethyl acetate in each time. The layer of ethyl acetate was washed with saturated aqueous sodium chloride solution. After the layer was dehydrated with anhydrous magnesium sulfate the ethyl acetate was evaporated and the residue dried. The residue thus obtained was pulverized by adding ether thereto to give powdery solid. The solid was filtered and was washed with ether and then dried to give 2.1 g of 7-(D-2-formyloxy-2-phenylacetoamide)-3-(1-methyl-1H-tetrazol-5-ylthiomethyl)-3-cephem-4-carboxylic acid at a yield of 85%. The analytical values were identical with those of Comparative Example 1.

Example 4

The solution consisting of 3.1 g (0.011 mole) of O-formyl-D-(—)-mandelic acid-1-methyl-1H-tetrazol-5-ylthiol ester and 20 ml of tetrahydrofuran (THF) was cooled at 5°C, and was added to 30 ml of 50% aqueous tetrahydrofuran solution containing 3.3 g (0.01 mole) of 7-amino-3-(1-methyl-1H-tetrazol-5-ylthiomethyl)-3-cephem-4-carboxylic acid and 1 g (0.01 mole) of triethylamine while the temperature of the solution was maintained at 5°C with stirring. The solution was further stirred at room temperature for 4 hours. Then the THF was removed from the solution under reduced pressure

and the solution was extracted twice with 25 ml of ethyl acetate in each time. After the extract was washed with 10 ml of saturated aqueous sodium hydrogencarbonate solution, the extract was added to the water layer separated at the extraction as described above. To the solution was added 6N hydrochloric acid to adjust the pH of the solution to 2.5, and the solution was extracted three times with 50 ml of ethyl acetate in each time. The ethyl acetate layer was washed with 15 ml of water and deydrated with anhydrous magnesium sulfate and then ethyl acetate was evaporated and the residue dried to give 4.4 g of 7-(D-2-formyloxy-2-phenylacetoamido)-3-(1-methyl-1H-tetrazol-5-ylthiomethyl)-3-cephem-4-carboxylic acid at the yield of 90%.

In 40 ml of water containing 3.2 g of sodium carbonate were dissolved 3.5 g of 7-(D-2-formyloxy-2-phenylacetoamido)-3-(1-methyl-1H-tetrazol-5-ylthiomethyl)-3-cephem-4-carboxylic acid thus obtained, and the solution was stirred at room temperature for 3 hours. Then the solution was diluted with 20 ml mixture of ice and water and added with 6N hydrochloric acid to adjust the pH of the solution to 2.5, and was extracted three times with 50 ml of ethyl acetate in each time. The layer of ethyl acetate was washed twice with 20 ml of water in each time and dehydrated with anhydrous magnesium sulfate, and then ethyl acetate was evaporated and the residue dried to give 7-D-mandelamido-3-(1-methyl-1H-tetrazol-5-ylthiomethyl)-3-cephem-4-carboxylic acid (cefamandole) in the form of yellow foam.

NMR Spectrum ($D_2O$), $\delta$ ppm:

3.50 (s, 2H), 3.92 (s, 3H), 4.20 (s, 2H), 5.04 (d, 1H), 5.24 (s, 1H), 5.64 (d, 1H), 7.44 (s, 5H)

Thus obtained 7-D-mandelamido-3-(1-methyl-1H-tetrazol-5-ylthiomethyl)-3-cephem-4-carboxylic acid (cefamandole) was dissolved in 50 ml of anhydrous ethanol and the solution was filtered. To the filtrate was added dropwise 6 ml of absolute methanol containing 1.09 g (0.008 mole) of sodium acetate trihydrates with stirring to give sodium cefamandole in the form of condensed slurry. The slurry was stirred at 0°C to 5°C for 30 minutes. The sodium cepfamandole was collected by filtration and washed with ethanol and then dried in vacuum to give 2.4 g of the compound at the yield of 51%.

Melting Point:

133—135°C

Ultraviolet Spectrum (Phosphoric acid buffer solution of pH 6.4):

$\lambda$ max=269 m$\mu$

Example 5

In a 3 l three neck distillation flask equipped with a thermometer were charged 36.2 g of 7-amino-3-(benzimidazol-2-ylthiomethyl)-3-cephem-4-carboxylic acid, 1 l of N,N-dimethylformamide and 1 l of water, and the mixed solution was stirred under heating at 60°C. Then, to the solution were added 30.0 g of D-(—)-mandelic acid-1-methyl-1H-tetrazol-5-ylthiol ester and 100 ml of 0.5 N hydrochloric acid as a catalyst. The reaction was carried out at 60°C for 3 hours with stirring. The reaction rate of the reaction solution was measured by high performance liquid chromatography and found to be 93%. The reaction solution was cooled and washed with ether by the conventional method. After the solution was added with 1N hydrochloric acid to adjust the pH of the solution to 1.5, the solution was extracted three times with 2 l of ethyl acetate in each time. The extract was dried with anhydrous magnesium sulfate and the solvent was removed from the extract under reduced pressure to give 33.1 g of 7-D-mandelamido-3-(1-methyl-1H-tetrazol-5-ylthiomethyl)-3-cephem-4-carboxylic acid in the form of white powder having a purity of 97%. The analytical values were identical with those of Example 4.

Comparative Example 2

In a 3 l three neck distillation flask equipped with a thermometer were charged 27.2 g of 7-aminocephalosporanic acid, 1.1 l of acetone and 1 l of water, and the mixed solution was stirred under heating at 60°C. Then, to the solution was added 30.0 g of mandelic acid-1-methyl-tetrazol-2-yl-thiol ester, and the reaction solution was stirred at 60°C for 4 hours. The reaction rate of the solution was measured by high performance liquid chromatography and was found to be 53%. The reaction solution was cooled and washed with ether by the conventional method. After the solution was added with 1N hydrochloric acid to adjust the pH of the solution to 1.5, the solution was extracted three times with 2 l of ethyl acetate in each time. The extract was dried with anhydrous magnesium sulfate and the solvent was removed from the extract under reduced pressure to give 18.4 g of 7-D-mandelamido-3-(1-methyl-1H-tetrazol-5-ylthiomethyl)-3-cephem-4-carboxylic acid in the form of white powder at purity of 98%. The analytical values were identical with those of Example 4.

Example 6

The solution consisting of 2.8 g (0.011 mole) of D-(—)-mandelic acid-1-methyl-1H-tetrazol-5-ylthiol ester and 20 ml of tetrahydrofuran (THF) was cooled at 5°C. The solution was added to 30 ml of 50% tetrahydrofuran aqueous solution containing 3.3 g (0.01 mole) of 7-amino-3-(1-methyl-1H-tetrazol-5-ylthiomethyl)-3-cephem-4-carboxylic acid and 1 g (0.01 mole) of triethylamine at 5°C with

stirring. The stirring was continued for 4 hours at room temperature. THF was removed from the solution under reduced pressure and the solution was extracted twice with 25 ml of ethyl acetate in each time. After the extract was washed with 10 ml of saturated aqueous sodium hydrogencarbonate solution, the extract was added to the water layer separated at the extraction as described above. To the solution was added 6N hydrochloric acid to adjust the pH of the solution to 2.5, and the solution was extracted three times with 50 ml of ethyl acetate in each time. The ethyl acetate layer was washed with 15 ml of water and dehydrated with anhydrous magnesium sulfate and then ethyl acetate was evaporated and the residue dried to give 3.5 g of 7-D-mandelamido-3-(1-methyl-1H-tetrazol-5-ylthiomethyl)-3-cephem-4-carboxylic acid (cefamandole). The analytical values were identical with those of Example 4.

**Claims for the Contracting States: BE CH DE FR GB IT LI LU NL SE**

1. A tetrazole-5-thiol ester having D-configuration of the formula:

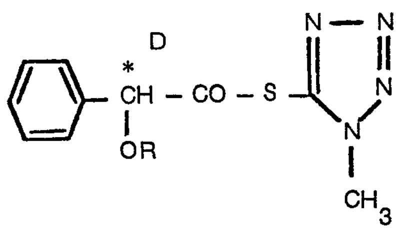

(I)

wherein R is a hydrogen atom or a formyl group.

2. The tetrazole-5-thiol ester of Claim 1, wherein R is a hydrogen atom.

3. The tetrazole-5-thiol ester of Claim 1, wherein R is a formyl group.

4. A process for preparing the tetrazole-5-thiol ester of Claim 1, which comprises reacting
(1) a 1-methyl-1H-tetrazole-5-thiol,
(2) its sodium or potassium salts,
(3) its reaction products of trimethylaluminum or triethylaluminum,
(4) its trimethylsilyl derivative and the triethylsilyl derivative or
(5) its trifluoroacetates with
(1) D-(—)-mandelic acid or O-formyl-D-(—)-mandelic acid (in the presence of a dehydrating agent in case of a reaction with 1-methyl-1H-tetrazole-5-thiol),
(2) the acid halides thereof,
(3) the mixed acid anhydrides thereof,
(4) the acid amides thereof,
(5) the esters thereof,
(6) the acid azides thereof or
(7) the nitriles thereof.

5. A process for preparing a cefamandole having D-configuration in the side chain of the formula:

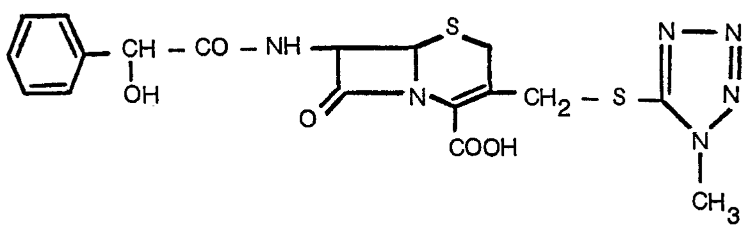

(II)

or its salt which comprises reacting a D-(—)-mandeloyl-1-methyl-1H-tetrazol-5-ylthiol ester of the formula,

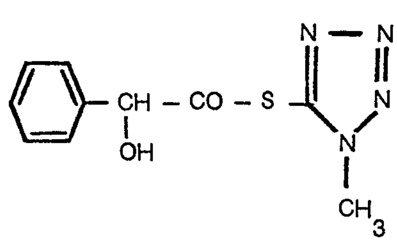

(Ia)

with a compound of the formula:

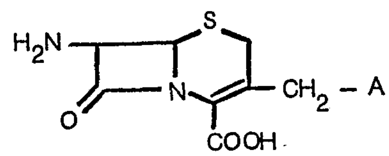

(IIIa)

wherein A is a benzimidazol-2-ylthio group or a 1-methyl-1H-tetrazol-5-ylthio group, or its salt.

6. A process for preparing a cefamandole having D-configuration in a side chain of the formula:

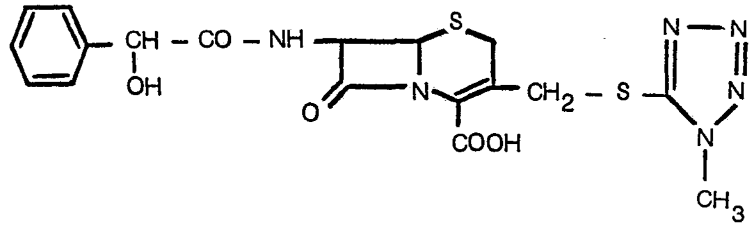

(II)

which comprises reacting a D-(—)-mandeloyl-1-methyl-1H-tetrazol-5-ylthiol ester of the formula:

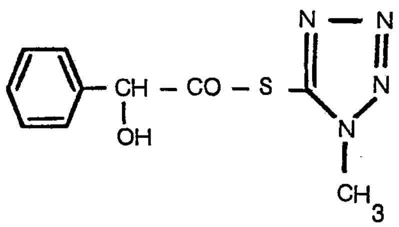

(Ia)

with a compound of the formula:

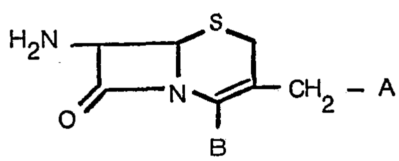

(IIIb)

wherein A is a benzimidazol-2-ylthio group or a 1-methyl-1H-tetrazol-5-ylthio group and B is a carboxylic acid ester group, to produce a compound of the formula:

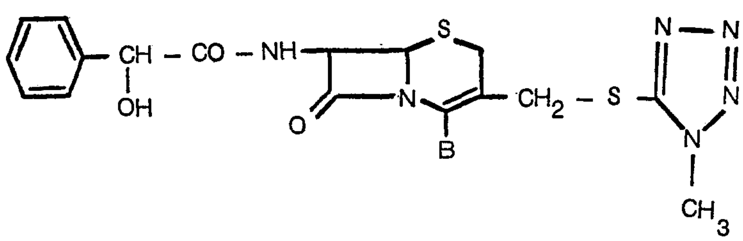

(IIa)

wherein B is a carboxylic acid ester group, and then cleaving the ester group of the compound (IIa).

7. A process for preparing a cefamandole having D-configuration in the side chain of the formula:

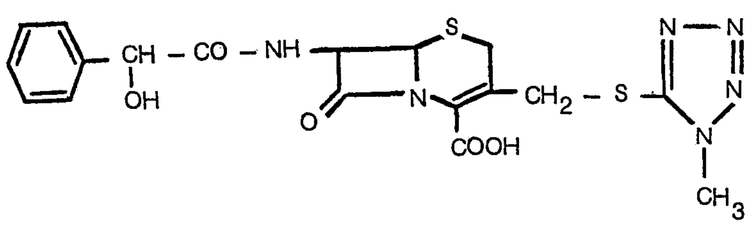

(II)

which comprises reacting a O-formyl-D-(—)-mandeloyl-1-methyl-1H-tetrazol-5-ylthiol ester of the formula:

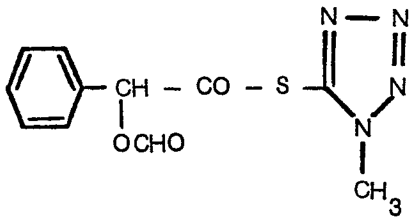

(Ib)

with a compound of the formula:

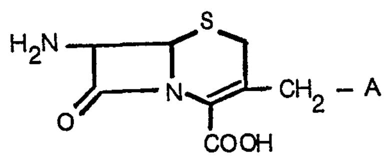

(IIIa)

wherein A is a benzimidazol-2-ylthio group or a 1-methyl-1H-tetrazol-5-ylthio group, or its salt to produce a compound of the formula:

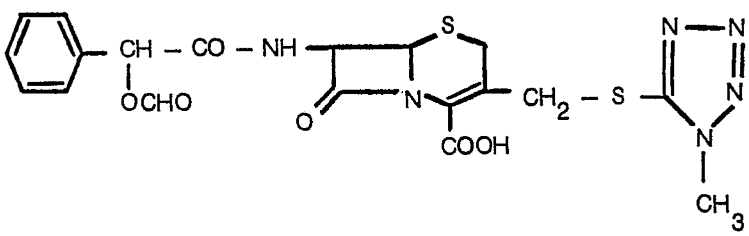

(IIb)

or its salt,
and treating the compound of the formula (IIb) with an alkaline solution.

8. A process for preparing a cefamandole having D-configuration in the side chain of the formula:

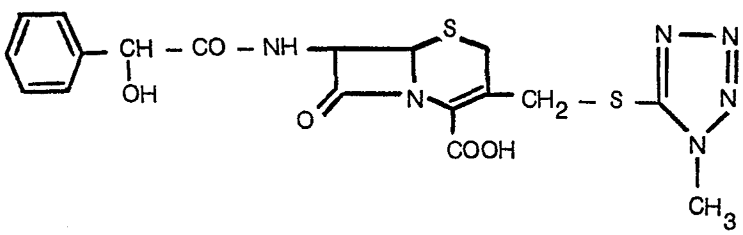

(II)

which comprises reacting a O-formyl-D-(—)-mandeloyl-1-methyl-1H-tetrazol-5-ylthiol ester of the formula:

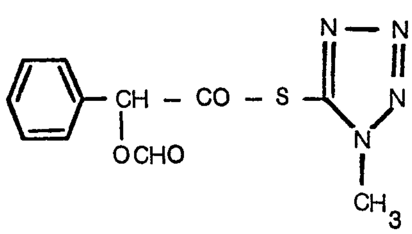

(Ib)

with a compound of the formula:

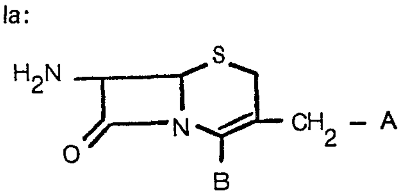

(IIIb)

wherein A is a benzimidazol-2-ylthio group or a 1-methyl-1H-tetrazol-5-ylthio group and B is a carboxylic acid ester group, to produce a compound of the formula:

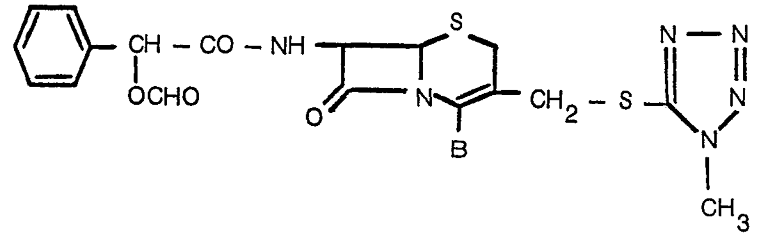

(IIc)

wherein B is a carboxylic acid ester group, cleaving the ester group of the compound (IIc) and then treating the compound (IIc) with an alkaline solution.

9. The process according to Claims 5, 6, 7 and 8, wherein the reaction is carried out in the presence of a solvent.

10. The process of Claims 5—8, wherein the salt of the compounds of the formulae (II) and (IIIa) is selected from alkali metals, alkaline earth metals and nitrogen-containing organic bases.

11. The process of Claim 10, wherein the salt is selected from sodium, potassium, calcium, magnesium, trimethylamine, triethylamine, pyridine, N-methylpiperidine and N-methylmorpholine.

12. The process of Claims 5—8, wherein the reactions are conducted at a pH of from 1 to 10.

13. The process of Claims 5—8, wherein A is a benzimidazol-2-ylthio group.

14. The process of Claims 5—8, wherein A is a 1-methyl-1H-tetrazol-5-ylthio group.

15. The process of Claims 5—8, wherein the reaction of the compound (Ia) or (Ib) and the compound (IIIa) or (IIIb) is conducted by using a catalyst of an inorganic acid or an organic acid.

16. The process of Claim 15, wherein the inorganic acid is hydrochloric acid or sulfuric acid.

17. The process of Claim 15, wherein the organic acid is 1-methyl-1H-tetrazole-5-thiol.

**Claims for the Contracting State: AT**

1. A process for preparing a tetrazole-5-thiol ester having D-configuration of the formula:

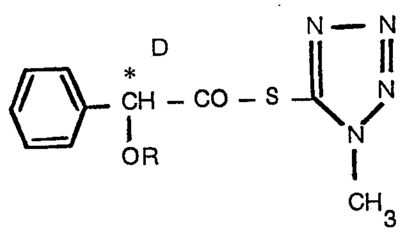

(I)

wherein R is a hydrogen atom or a formyl group, which comprises reacting
(1) a 1-methyl-1H-tetrazole-5-thiol,
(2) its sodium or potassium salts,
(3) its reaction products of trimethylaluminum or triethylaluminum,
(4) its trimethylsilyl derivative and the triethylsilyl derivative or
(5) its trifluoroacetates with
(1) D-(—)-mandelic acid or O-formyl-D-(—)-mandelic acid (in the presence of a dehydrating agent in case of a reaction with 1-methyl-1H-tetrazole-5-thiol),
(2) the acid halides thereof,
(3) the mixed acid anhydrides thereof,
(4) the acid amides thereof,
(5) the esters thereof,
(6) the acid azides thereof or
(7) the nitriles thereof.

2. A process of Claim 1, wherein O-formyl-D-(—)-mandelic acid or its derivative (2) to (7) according to Claim 1 is used.

3. A process for preparing a cefamandole having D-configuration in the side chain of the formula:

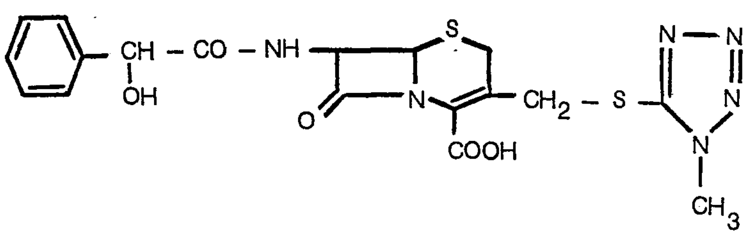

(II)

or its salt which comprises reacting a D-(—)-mandeloyl-1-methyl-1H-tetrazol-5-ylthiol ester of the formula,

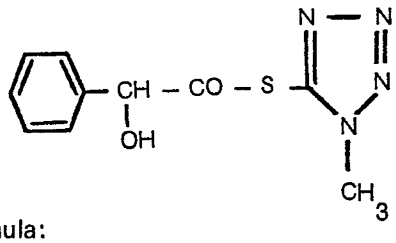

(Ia)

with a compound of the formula:

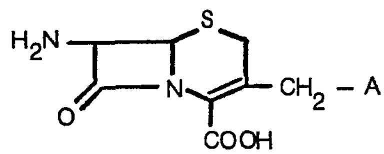

(IIIa)

wherein A is a benzimidazol-2-ylthio group or a 1-methyl-1H-tetrazol-5-ylthio group, or its salt.

4. A process for preparing a cefamandole having D-configuration in a side chain of the formula:

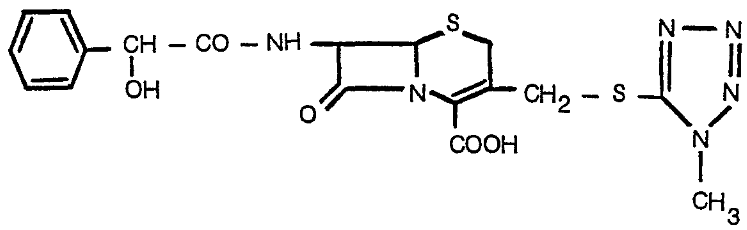

(II)

which comprises reacting a D-(—)-mandeloyl-1-methyl-1H-tetrazol-5-ylthiol ester of the formula:

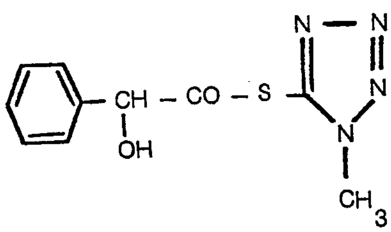

(Ia)

with a compound of the formula:

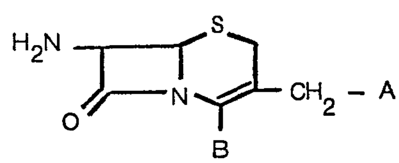

(IIIb)

wherein A is a benzimidazol-2-ylthio group or a 1-methyl-1H-tetrazol-5-ylthio group and B is a carboxylic acid ester group, to produce a compound of the formula:

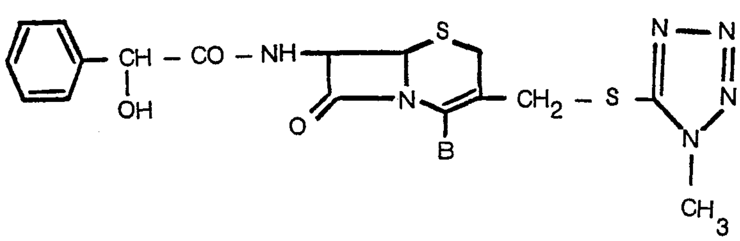

(IIa)

wherein B is a carboxylic acid ester group, and then cleaving the ester group of the compound (IIa).

5. A process for preparing a cefamandole having D-configuration in the side chain of the formula:

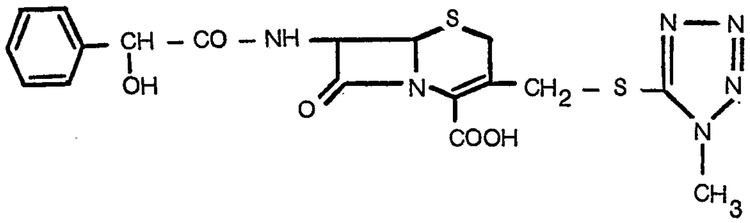

(II)

or its salt which comprises reacting a O-formyl-D-(—)-mandeloyl-1-methyl-1H-tetrazol-5-ylthiol ester of the formula:

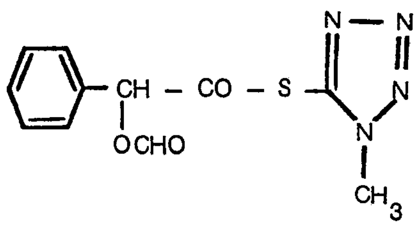

(Ib)

with a compound of the formula:

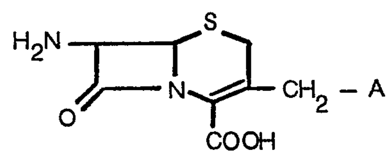

(IIIa)

wherein A is a benzimidazol-2-ylthio group or a 1-methyl-1H-tetrazol-5-ylthio group, or its salt to produce a compound of the formula:

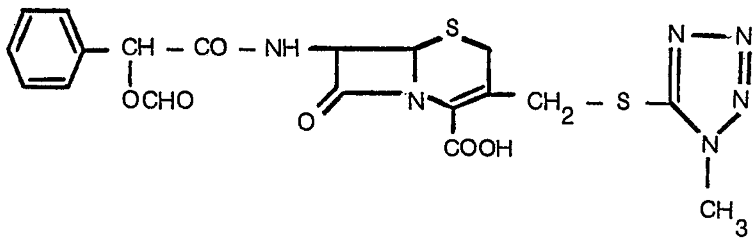

(IIb)

or its salt, and treating the compound of the formula (IIb) with an alkaline solution.

6. A process for preparing a cefamandole having D-configuration in a side chain of the formula:

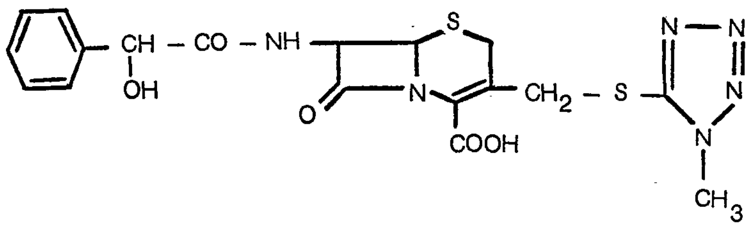

(II)

which comprises reacting a O-formyl-D-(—)-mandeloyl-1-methyl-1H-tetrazol-5-ylthiol ester of the formula:

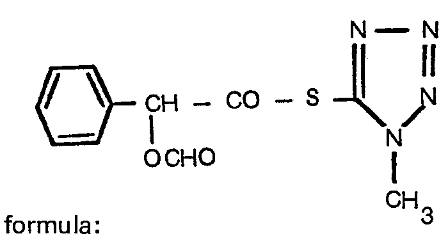

(Ib)

with a compound of the formula:

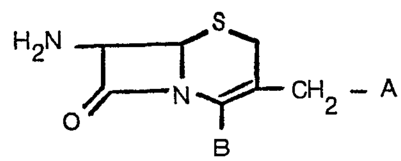

(IIIb)

wherein A is a benzimidazol-2-ylthio group or a 1-methyl-1H-tetrazol-5-ylthio group and B is a carboxylic acid ester group, to produce a compound of the formula:

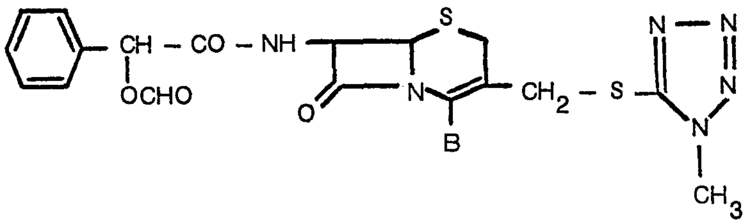

(IIc)

wherein B is a carboxylic acid ester group, cleaving the ester group of the compound (IIc) and then treating the compound (IIc) with an alkaline solution.

7. The process according to Claims 3 to 6, wherein the reaction is carried out in the presence of a solvent.

8. The process of Claims 3 to 6, wherein the salt of the compounds of the formulae (II) and (IIIa) is selected from alkali metals, alkaline earth metals and nitrogen containing organic bases.

9. The process of Claim 8, wherein the salt is selected from sodium, potassium, calcium, magnesium, trimethylamine, triethylamine, pyridine, N-methylpiperidine and N-methyl-morpholine.

10. The process of Claims 3 to 6, wherein the reactions are conducted at a pH of from 1 to 10.

11. The process of Claims 3 to 6, wherein A is a benzimidazol-2-ylthio group.

12. The process of Claims 3 to 6, wherein A is a 1-methyl-1H-tetrazole-5-ylthio group.

13. The process of Claims 3 to 6, wherein the reaction of the compound (Ia) or (Ib) and the compound (IIIa) or (IIIb) is conducted by using a catalyst of an inorganic acid or an organic acid.

14. The process of Claim 13, wherein the inorganic acid is hydrochloric acid or sulfuric acid.

15. The process of Claim 13, wherein the organic acid is 1-methyl-1H-tetrazole-5-thiol.

**Revendications pour les Etats contractants: BE CH DE FR GB IT LU NL SE LI**

1. Tétrazole-5-thiol ester ayant la configuration D de formule

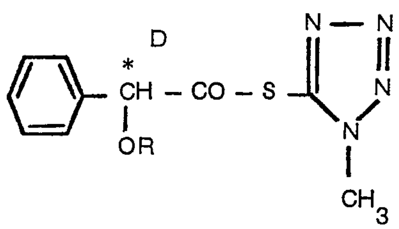

(I)

où R est un atome d'hydrogène ou un groupe formyle.

2. Tétrazole-5-thiol ester selon la revendication 1 où R est un atome d'hydrogène.

3. Tétrazole-5-thiol ester selon la revendication 1 où R est un groupe formyle.

4. Procédé de préparation du tétrazole-5-thiol ester selon la revendication 1 qui consiste à faire réagir

(1) un 1-méthyl-1H-tétrazole-5-thiol,
(2) ses sels de sodium ou de potassium,
(3) ses produits réactionnels de triméthyl-aluminium ou triéthyl-aluminium,
(4) son dérivé de triméthylsilyle et le dérivé de triéthylsilyl ou
(5) ses trifluoroacétates avec
(1) de l'acide D-(—)-mandélique ou de l'acide O-formyl-D-(—)-mandélique (en présence d'un agent déshydratant dans le cas d'une réaction avec du 1-méthyl-1H-tétrazole-5-thiol),
(2) ses halogénures d'acide,
(3) ses anhydrides d'acide mélangés,
(4) ses amides d'acide,

(5) ses esters,
(6) ses azides d'acide ou
(7) ses nitriles.

5. Procédé de préparation de céfamandole ayant la configuration D dans la chaîne secondaire de formule

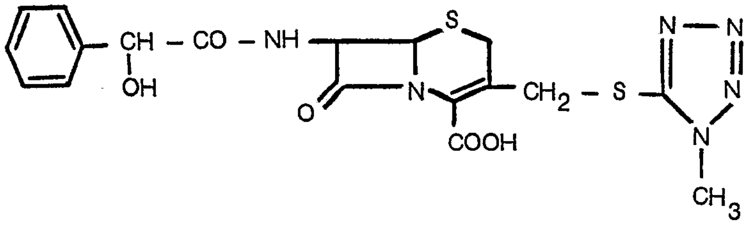

(II)

ou son sel qui consiste à faire réagir un D-(—)-mandéloyl-1-méthyl-1H-tétrazole-5-ylthiol ester de formule

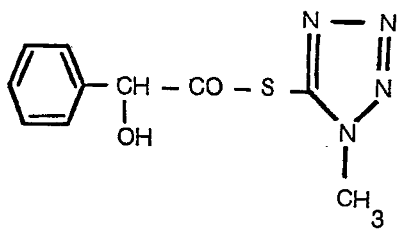

(Ia)

avec un composé de formule

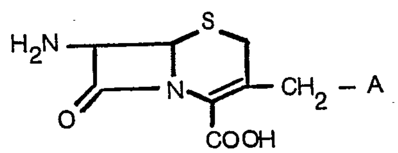

(IIIa)

où A est un groupe benzimidazole-2-ylthio ou un groupe 1-méthyl-1H-tétrazole-5-ylthio ou son sel.

6. Procédé de préparation d'un céfamandole ayant la configuration D dans une chaîne secondaire de formule

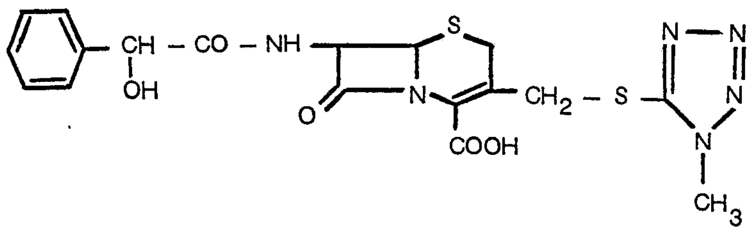

(II)

qui consiste à faire réagir un D-(—)-mandéloyl-1-méthyl-1H-tétrazole-5-ylthiol ester de formule:

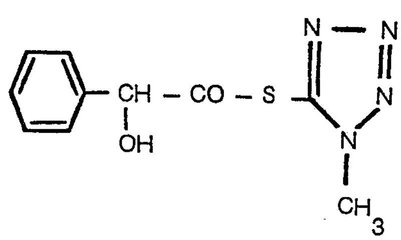

(Ia)

avec un composé de formule

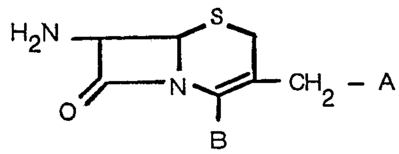

(IIIb)

où A est un groupe benzimidazole-2-ylthio ou un groupe 1-méthyl-1H-tétrazole-5-ylthio et B est un groupe ester d'acide carboxylique pour produire un composé de formule

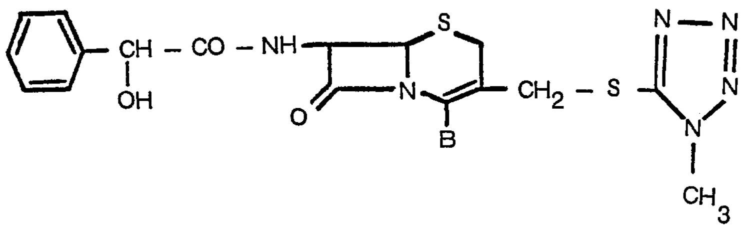

(IIa)

où B est un groupe ester d'acide carboxylique et à scinder ensuite le groupe ester du composé (IIa).

7. Procédé de préparation d'un céfamandole ayant la configuration D dans la chaîne secondaire de formule

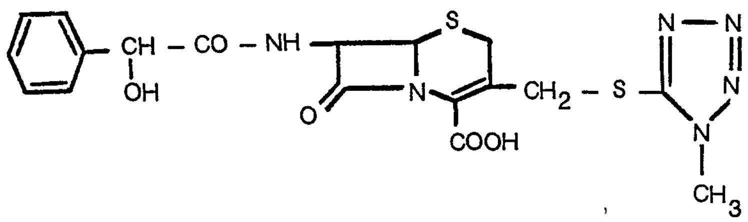

(II)

qui consiste à faire réagir un O-formyl-D-(—)-mandéloyl-1-méthyl-1H-tétrazole-5-ylthiol ester de formule:

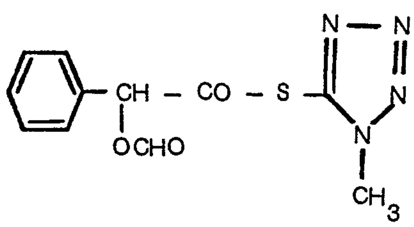

(Ib)

avec un composé de formule

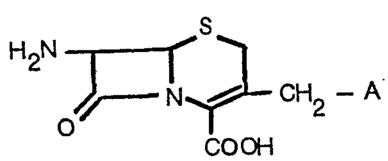

(IIIa)

où A est un groupe benzimidazole-2-ylthio ou un groupe 1-méthyl-1H-tétrazole-5-ylthio ou son sel, pour produire un composé de formule

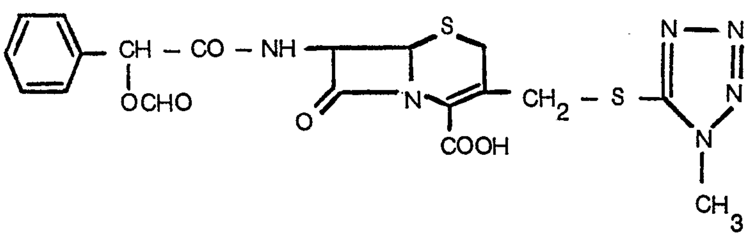

(IIb)

ou son sel,
et à traiter le composé de formule (IIb) avec une solution alcaline.

8. Procédé de préparation d'un céfamandole ayant la configuration D dans la chaîne secondaire de formule

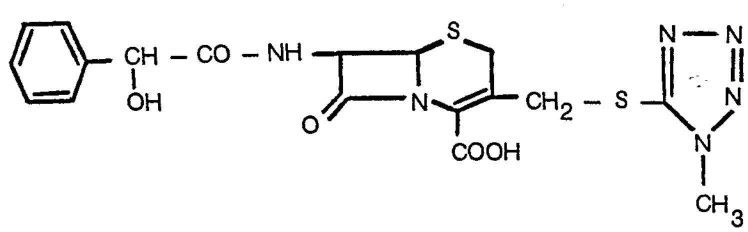

(II)

qui consiste à faire réagir un O-formyl-D-(—)-mandéloyl-1-méthyl-1H-tétrazole-5-ylthiol ester de formule

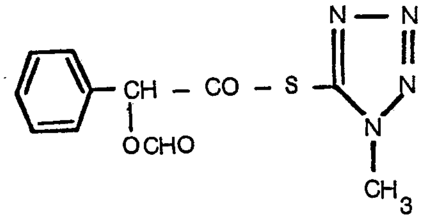

(Ib)

avec un composé de formule

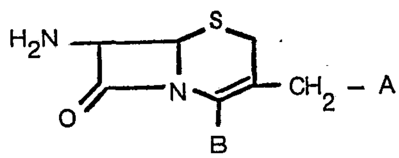

(IIIb)

où A est un groupe benzimidazole-2-ylthio ou un groupe 1-méthyl-1H-tétrazole-5-ylthio et B est un groupe ester d'acide carboxylique, pour produire un composé de formule

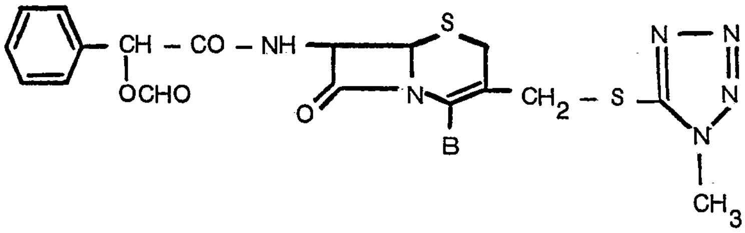

(IIc)

où B est un groupe ester d'acide carboxylique, à scinder le groupe ester du composé (IIc) puis à traiter le composé (IIc) avec un solution alcaline.

9. Procédé selon les revendications 6, 6, 7 et 8, où la réaction est mise en oeuvre en présence d'un solvant.

10. Procédé selon la revendications 5 à 8, où le sel des composés des formules (II) et (IIIa) est choisi parmi des métaux alcalins, des métaux alcalino-terreux et des bases organiques contenant de l'azote.

11. Procédé selon la revendication 10, où le sel est choisi parmi le sodium, le potassium, le calcium, le magnésium, la triméthylamine, la triéthylamine, la pyridine, la N-méthylpiperidine et la N-méthylmorpholine.

12. Procédé selon les revendications 5 à 8, où les réactions sont entreprises à un pH compris entre 1 et 10.

13. Procédé selon les revendications 5 à 8, où A est un groupe benzimidazole-2-ylthio.

14. Procédé selon les revendications 5 à 8, où A est un groupe 1-méthyl-1H-tétrazole-5-ylthio.

15. Procédé selon les revendications 5 à 8, où la réaction des composé (Ia) ou (Ib) et du composé (IIIa) ou (IIIb) est entreprise en utilisant un catalyseur d'un acide inorganique ou d'un acide organique.

16. Procédé selon la revendication 15, où l'acide inorganique est l'acide chlorhydrique ou l'acide sulfurique.

17. Procédé selon la revendication 15, où l'acide organique est du 1-méthyl-1H-tétrazole-5-thiol.

**Revendications pour l'Etat contractant: AT**

1. Procédé de préparation de tétrazole-5-thiol ester ayant la configuration D de formule

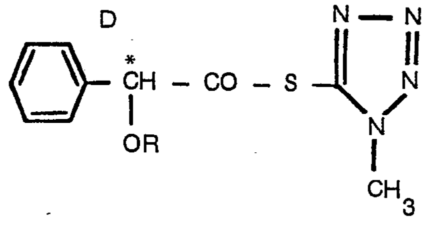

(I)

où R est un atome d'hydrogène ou un groupe formyle, qui consiste à faire réagir

(1) un 1-méthyl-1H-tétrazole-5-thiol,
(2) ses sels de sodium ou de potassium,
(3) ses produits réactionnels de triméthyl-aluminium ou triéthyl-aluminium,
(4) son dérivé de triméthylsilyle ou son dérivé de triéthylsilyle ou
(5) ses trifluoroacétates avec
(1) de l'acide D-(—)-mandélique ou de l'acide O-formyl-D-(—)-mandélique (en présence d'un agent déshydratant dans le cas d'une réaction avec du 1-méthyl-1H-tétrazole-5-thiol),
(2) ses halogénures d'acide,
(3) ses anhydrides d'acide mélangés,
(4) ses amides d'acide,
(5) ses esters,
(6) ses azides d'acide ou
(7) ses nitriles.

2. Procédé selon la revendication 1, où de l'acide O-formyl-D-(—)-mandélique ou son dérivé (2) à (7) selon la revendication 1 est utilisé.

3. Procédé de préparation de céfamandole ayant la configuration D dans la chaîne secondaire de formule

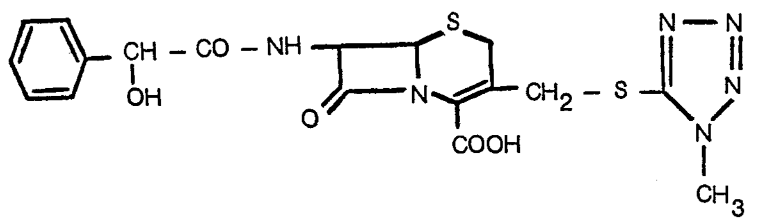

(II)

ou son sel qui consiste à faire réagir un D-(—)-mandéloyl-1-méthyl-1H-tétrazole-5-ylthiol ester de formule

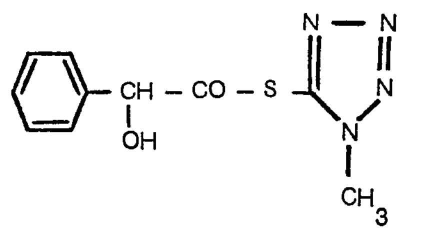

(Ia)

avec un composé de formule

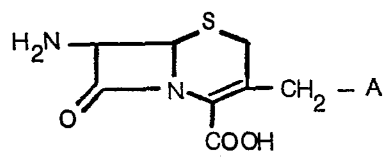

(IIIa)

où A est un groupe benzimidazole-2-ylthio ou un groupe 1-méthyl-1H-tétrazole-5-ylthio ou son sel.

4. Procédé de préparation d'un céfamandole ayant la configuration D dans une chaîne secondaire de formule

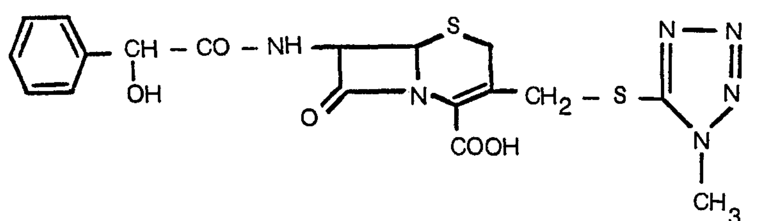

(II)

qui consiste à faire réagir un D-(—)-mandéloyl-1-méthyl-1H-tétrazole-5-ylthiol ester de formule:

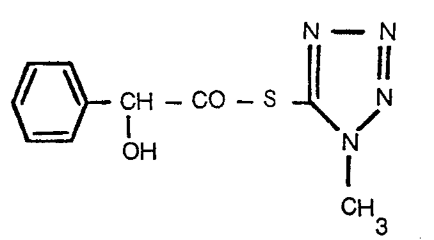

(Ia)

avec un composé de formule

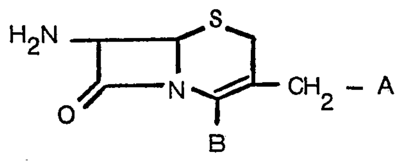

(IIIb)

où A est un groupe benzimidazole-2-ylthio ou un groupe 1-méthyl-1H-tétrazole-5-ylthio et B est un groupe ester d'acide carboxylique pour produire un composé de formule

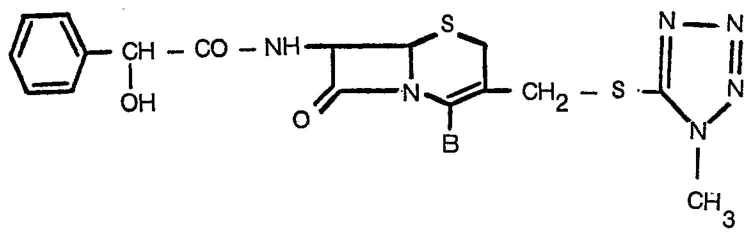

(IIa)

où B est un groupe ester d'acide carboxylique et à scinder ensuite le groupe ester du composé (IIa).

5. Procédé de préparation d'un céfamandole ayant la configuration D dans la chaîne secondaire de formule

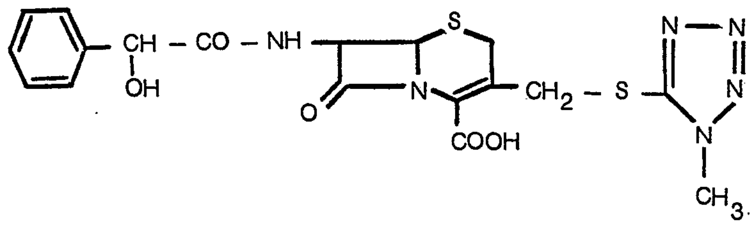

(II)

ou son sel qui consiste à faire réagir un O-formyl-D-(—)-mandéloyl-1-méthyl-1H-tétrazole-5-ylthiol ester de formule

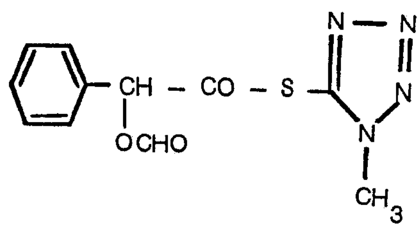

(Ib)

avec un composé de formule

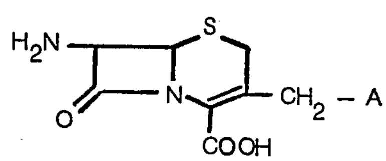

(IIIa)

où A est un groupe benzimidazole-2-ylthio ou un groupe 1-méthyl-1H-tétrazole-5-ylthio, ou son sel pour produire un composé de formule

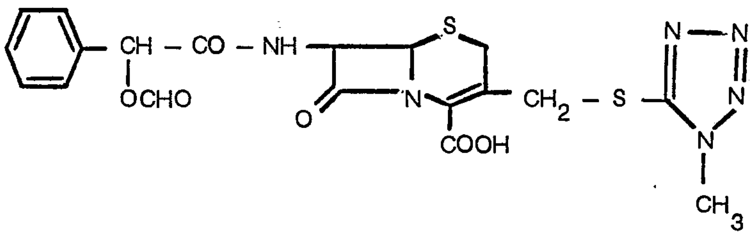

(IIb)

ou son sel,
et à traiter le composé de formule (IIb) avec une solution alcaline.

6. Procédé de préparation d'un céfamandole ayant la configuration D dans la chaîne secondaire de formule

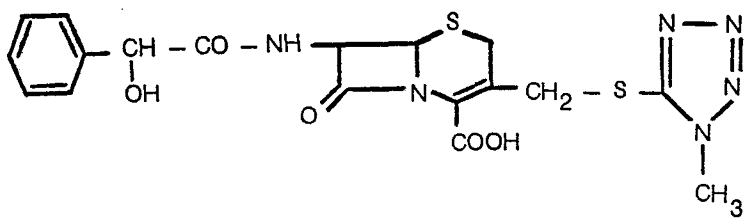

(II)

qui consiste à faire réagir un O-formyl-D-(—)-mandéloyl-1-méthyl-1H-tétrazole-5-ylthio ester de formule:

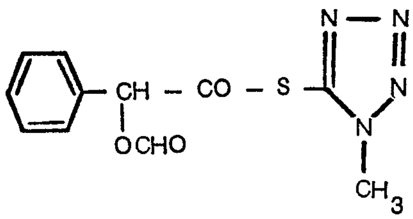

(Ib)

avec un composé de formule

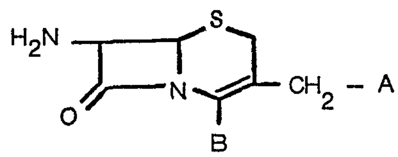

(IIIb)

où A est un groupe benzimidazole-2-ylthio ou un groupe 1-méthyl-1H-tétrazole-5-ylthio et B est un groupe ester d'acide carboxylique pour produire un composé de formule

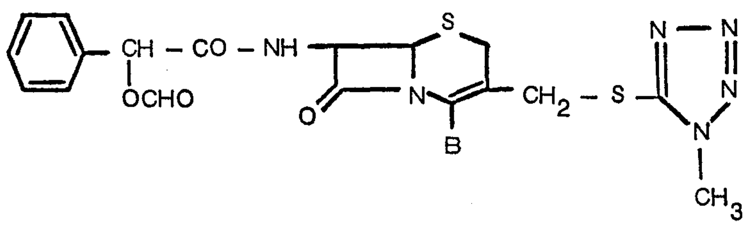

(IIc)

où B est un groupe ester d'acide carboxylique et à scinder le groupe ester du composé (IIc) puis à traiter le composé (IIc) avec une solution alcaline.

7. Procédé selon les revendications 3 à 6, où la réaction est mise en oeuvre en présence d'un solvant.

8. Procédé selon des revendications 3 à 6, où le sel des composés des formules (II) et (IIIa) est choisi parmi des métaux alcalins, des métaux alcalino-terreux et des bases organiques contenant de l'azote.

9. Procédé selon la revendication 8, où le sel est choisi parmi le sodium, le potassium, le calcium, le magnésium, la triméthylamine, la triéthylamine, la pyridine, la N-méthylpiperidine et la N-méthylmorpholine.

10. Procédé selon les revendications 3 à 6, où les réactions sont entreprises à un compris entre 1 et 10.

11. Procédé selon les revendications 3 à 6, où A est un groupe benzimidazole-2-ylthio.

12. Procédé selon les revendications 3 à 6, où A est un groupe 1-méthyl-1H-tétrazole-5-ylthio.

13. Procédé selon les revendications 3 à 6, où la réaction du composé (Ia) ou (Ib) et du composé (IIIa) ou (IIIb) est entreprise en utilisant un catalyseur d'un acide inorganique ou d'un acide organique.

14. Procédé selon la revendication 13, où l'acide inorganique est l'acide chlorhydrique ou l'acide sulfurique.

15. Procédé selon la revendication 13, où l'acide organique est du 1-méthyl-1H-tétrazole-5-thiol.

**Patentansprüche für die Vertragsstaaten BE CH DE FR GB IT LU NL SE LI**

1. Ein Tetrazol-5-thiolester mit D-Konfiguration der Formel:

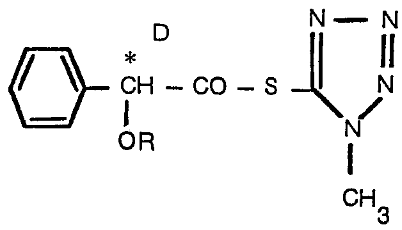

(I)

worin R eine Wasserstoffatom oder ein Formylrest ist.

2. Tetrazol-5-thiolester nach Anspruch 1, worin R ein Wasserstoffatom ist.

3. Tetrazol-5-thiolester nach Anspruch 1, worin R ein Formylrest ist.

4. Verfahren zur Herstellung des Tetrazol-5-thiolesters nach Anspruch 1, wobei man
(1) ein 1-Methyl-1H-tetrazol-5-thiol,
(2) seine Natrium- oder Kaliumsalze,
(3) seine Reaktionsprodukte mit Trimethylaluminium oder Triethylaluminium,
(4) sein Trimethylsilylderivat und das Triethylsilylderivat oder
(5) seine Trifluoracetate mit
(1) D-(—)-Mandelsäure oder O-Formyl-D-(—)-mandelsäure (in Gegenwart eines Dehydratisierungsmittels im Fall einer Reaktion mit 1-Methyl-1H-tetrazol-5-thiol),
(2) deren Säurehalogeniden,
(3) deren gemischten Säureanhydriden,
(4) deren Säureamiden,
(5) deren Estern,
(6) deren Säureaziden oder
(7) deren Nitrilen umsetzt.

5. Verfahren zur Herstellung eines Cefamandols mit D-Konfiguration in der Seitenkette der Formel:

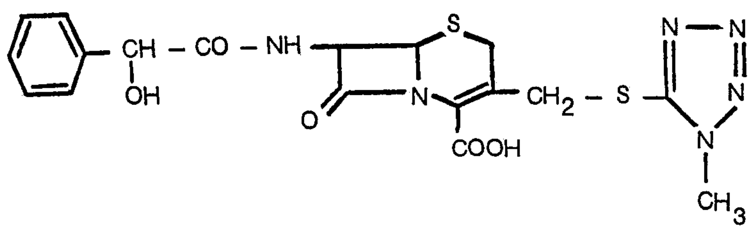

(II)

oder seines Salzes, wobei man einen D-(—)-Mandelsäure-1-methyl-1H-tetrazol-5-ylthiolester der Formel

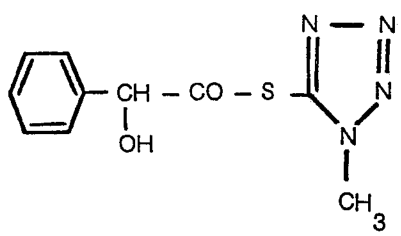

(Ia)

mit einer Verbindung der Formel:

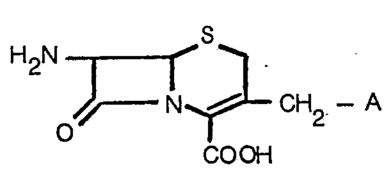

(IIIa)

worin A ein Benzimidazol-2-ylthiorest oder ein 1-Methyl-1H-tetrazol-5-ylthiorest ist, oder ihrem Salz umsetzt.

6. Verfahren zur Herstellung eines Cefamandols mit D-Konfiguration in einer Seitenkette der Formel:

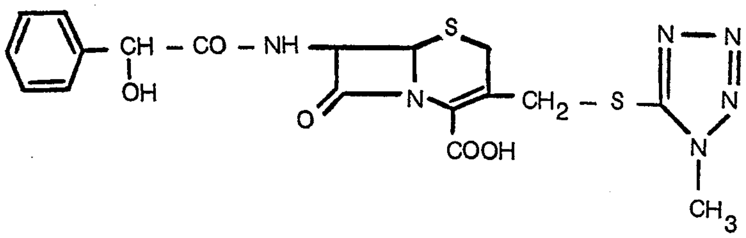

(II)

wobei man einen D-(—)-Mandelsäure-1-methyl-1H-tetrazol-5-ylthiolester der Formel:

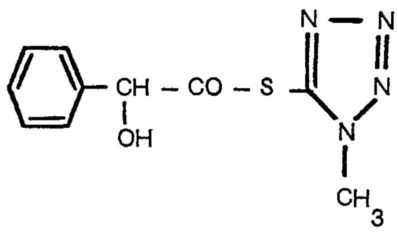

(Ia)

mit einer Verbindung der Formel umsetzt:

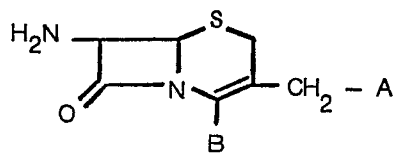

(IIIb)

worin A ein Benzimidazol-2-ylthiorest oder ein 1-Methyl-1H-tetrazol-5-ylthiorest ist und B ein Carbonsäureesterrest ist, um eine Verbindung der nachstehenden Formel herzustellen:

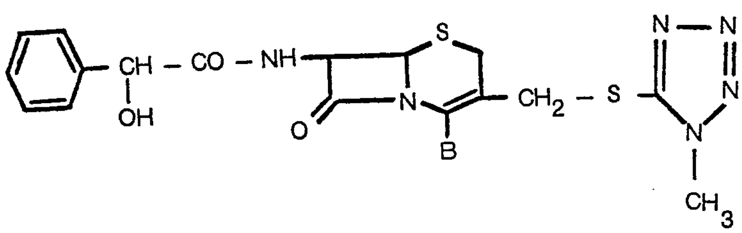

(IIa)

worin B ein Carbonsäureesterrest ist, und danach den Esterrest der Verbindung (IIa) abspaltet.

7. Verfahren zur Herstellung eines Cefamandols mit D-Konfiguration in der Seitenkette der Formel:

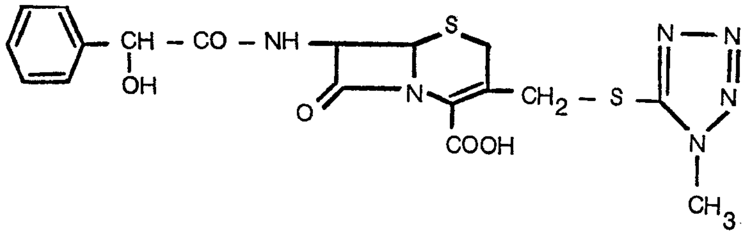

(II)

wobei man einen O-Formyl-D-(—)-mandelsäure-1-methyl-1H-tetrazol-5-ylthiolester der Formel:

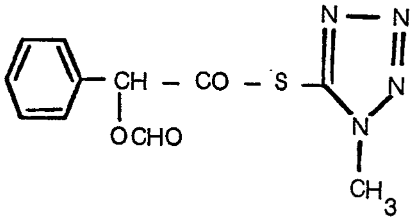

(Ib)

mit einer Verbindung der Formel:

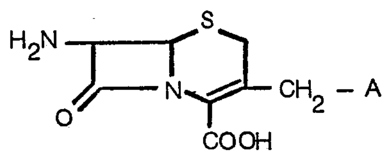

(IIIa)

worin A ein Benzimidazol-2-ylthiorest oder ein 1-Methyl-1H-tetrazol-5-ylthiorest ist, oder seinem Salz umsetzt, um eine Verbindung der Formel:

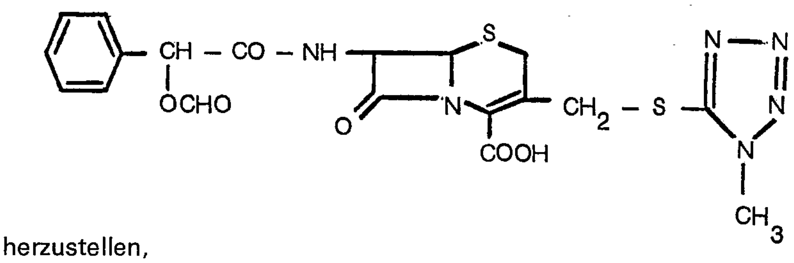

(IIb)

oder ihr Salz herzustellen,
und die Verbindung der Formel (IIb) mit einer alkalischen Lösung behandelt.

8. Verfahren zur Herstellung eines Cefamandols mit D-Konfiguration in der Seitenkette der Formel:

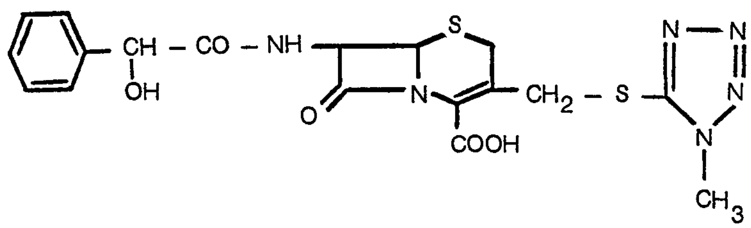

(II)

wobei man einen O-Formyl-D-(—)-mandelsäure-1-methyl-1H-tetrazol-5-ylthiolester der Formel:

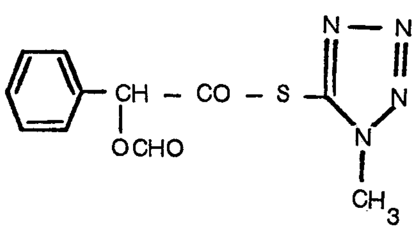

(Ib)

mit einer Verbindung der Formel umsetzt:

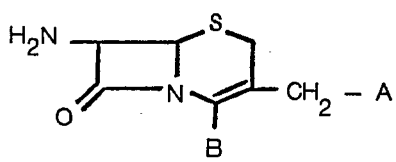

(IIIb)

worin A ein Benzimidazol-2-ylthiorest oder ein 1-Methyl-1H-tetrazol-5-ylthiorest ist und B ein Carbonsäureesterrest ist, um eine Verbindung der Formel herzustellen:

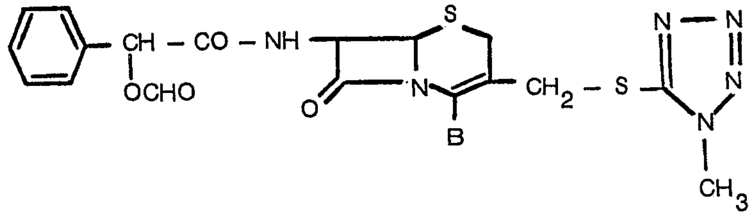

(IIc)

worin B ein Carbonsäureesterrest ist, den Esterrest der Verbindung (IIc) abspaltet und danach die Verbindung (IIc) mit einer alkalischen Lösung behandelt.

9. Verfahren nach den Ansprüchen 5, 6, 7 und 8, worin man die Reaktion in Gegenwart eines Lösungsmittels durchführt.

10. Verfahren nach den Ansprüchen 5 bis 8, worin man das Salz der Verbindungen der Formeln (II) und (IIIa) aus der aus Alkalimetallen, Erdalkalimetallen und stickstoffhaltigen organischen Basen bestehenden Gruppe auswählt.

11. Verfahren nach Anspruch 10, worin man das Salz aus der aus Natrium-, Kalium-, Calcium-, Magnesium-, Trimethylamin-, Triethylamin-, Pyridin-, N-Methylpiperidin- und N-Methylmorpholinsalz bestehenden Gruppe auswählt.

12. Verfahren nach den Ansprüchen 5 bis 8, worin man die Reaktionen bei eniem pH-Wert von 1 bis 10 durchführt.

13. Verfahren nach den Ansprüchen 5 bis 8, worin A ein Benzimidazol-2-ylthiorest ist.

14. Verfahren nach den Ansprüchen 5 bis 8, worin A ein 1-Methyl-1H-tetrazol-5-ylthiorest ist.

15. Verfahren nach den Ansprüchen 5 bis 8, worin man die Reaktion der Verbindung (Ia) oder (Ib) und der Verbindung (IIIa) oder (IIIb) durchführt, indem man einen Katalysator aus einer anorganischen Säure oder einer organischen Säure verwendet.

16. Verfahren nach Anspruch 15, worin die anorganische Säure Salzsäure oder Schwefelsäure ist.

17. Verfahren nach Anspruch 15, worin die organische Säure 1-Methyl-1H-tetrazol-5-thiol ist.

**Patentansprüche für den Vertragsstaat: AT**

1. Verfahren zur Herstellung eines Tetrazol-5-thiolesters mit D-Konfiguration der Formel:

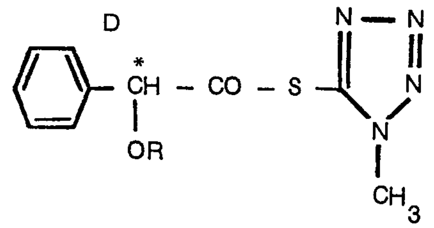

(I)

worin R ein Wasserstoffatom oder ein Formylrest ist, wobei man
(1) ein 1-Methyl-1H-tetrazol-5-thiol,
(2) seine Natrium- oder Kaliumsalze,
(3) seine Reaktionsprodukte mit Trimethylaluminium oder Triethylaluminium,
(4) sein Trimethylsilylderivat oder sein Triethylsilylderivat oder
(5) seine Trifluoracetate mit
(1) D-(—)-Mandelsäure oder O-Formyl-D-(—)-mandelsäure (in Gegenwart eines Dehydratisierungsmittels im Fall einer Reaktion mit 1-Methyl-1H-tetrazol-5-thiol),
(2) deren Säurehalogeniden,
(3) deren gemischten Säureanhydriden,
(4) deren Säureamiden,
(5) deren Estern,
(6) deren Säureaziden oder
(7) deren Nitrilen umsetzt.

2. Verfahren nach Anspruch 1, worin man O-Formyl-D-(—)-mandesäure oder ihr Derivat (2) bis (7) gemäß Anspruch 1 verwendet.

3. Verfahren zur Herstellung eines Cefamandols mit D-Konfiguration in der Seitenkette der Formel:

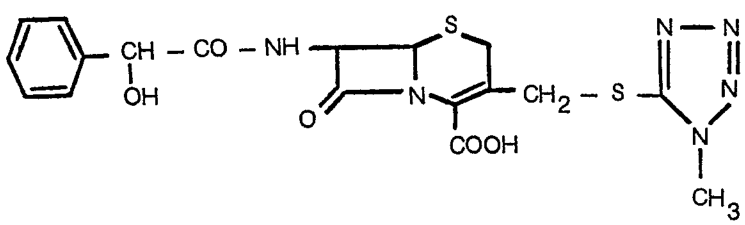

(II)

oder seines Salzes, wobei man einen D-(—)-Mandelsäure-1-methyl-1H-tetrazol-5-ylthiolester der Formel

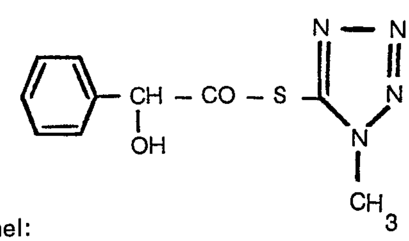

(Ia)

mit einer Verbindung der Formel:

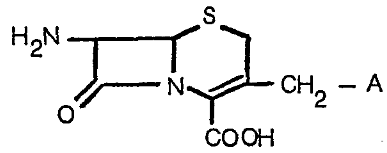

(IIIa)

worin A ein Benzimidazol-2-ylthiorest oder ein 1-Methyl-1H-tetrazol-5-ylthiorest ist, oder ihrem Salz umsetzt.

4. Verfahren zur Herstellung eines Cefamandols mit D-Konfiguration in einer Seitenkette der Formel:

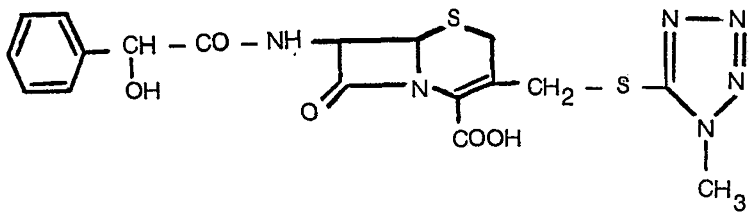

(II)

wobei man einen D-(—)-Mandelsäure-1-methyl-1H-tetrazol-5-ylthiolester der Formel:

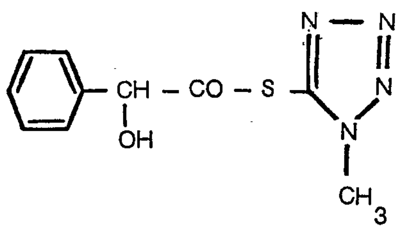

(Ia)

mit einer Verbindung der Formel umsetzt:

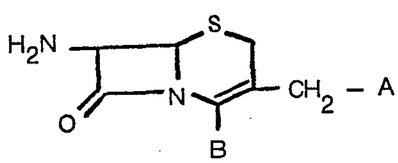

(IIIb)

worin A ein Benzimidazol-2-ylthiorest oder ein 1-Methyl-1H-tetrazol-5-ylthiorest ist und B ein Carbonsäureesterrest ist, um eine Verbindung der nachstehenden Formel herzustellen:

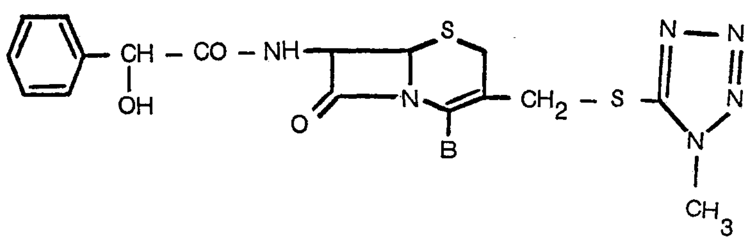

(IIa)

worin B ein Carbonsäureesterrest ist, und danach den Esterrest der Verbindung (IIa) abspaltet.

5. Verfahren zur Herstellung eines Cefamandols mit D-Konfiguration in der Seitenkette der Formel:

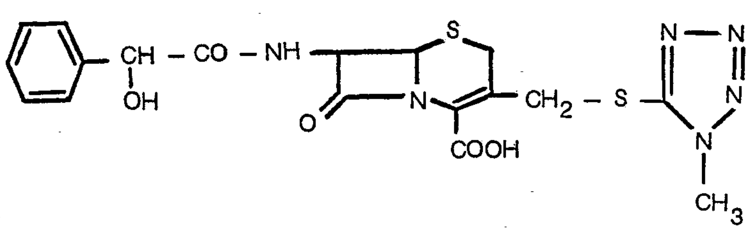

(II)

oder seinem Salz

wobei man einen O-Formyl-D-(—)-mandelsäure-1-methyl-1H-tetrazol-5-ylthiolester der Formel:

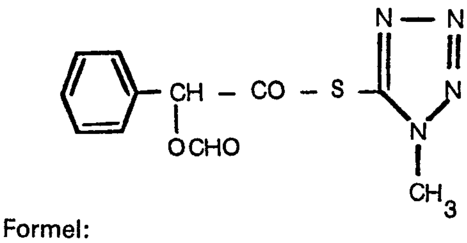

(Ib)

mit einer Verbindung der Formel:

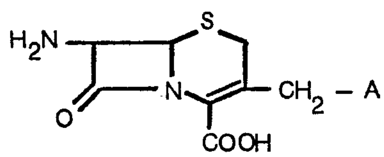

(IIIa)

worin A ein Benzimidazol-2-ylthiorest oder ein 1-Methyl-1H-tetrazol-5-ylthiorest ist, oder seinem Salz umsetzt, um eine Verbindung der Formel:

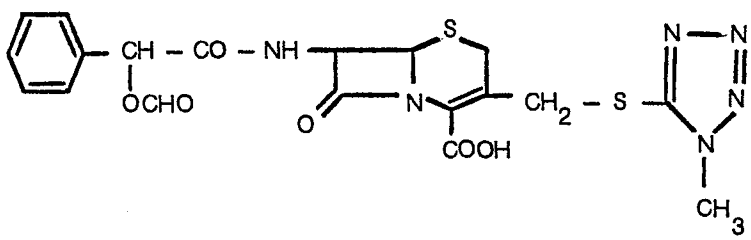

(IIb)

oder ihr Salz herzustellen,
und die Verbindung der Formel (IIb) mit einer alkalischen Lösung behandelt.

6. Verfahren zur Herstellung eines Cefamandole mit D-Konfiguration in der Seitenkette der Formel:

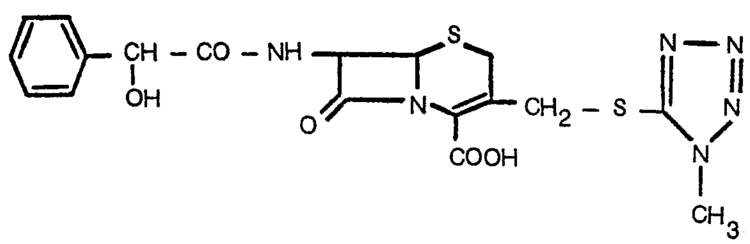

(II)

wobei man einen O-Formyl-D-(—)-mandelsäure-1-methyl-1H-tetrazol-5-ylthiolester der Formel:

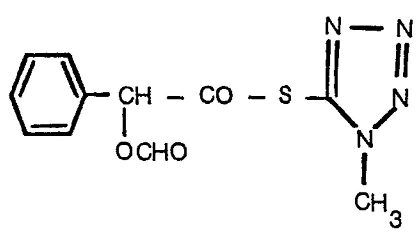

(Ib)

mit einer Verbindung der Formel umsetzt:

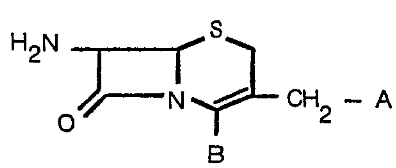

(IIIb)

worin A ein Benzimidazol-2-ylthiorest oder ein 1-Methyl-1H-tetrazol-5-ylthiorest ist und B ein Carbonsäureesterrest ist, um eine Verbindung der Formel herzustellen:

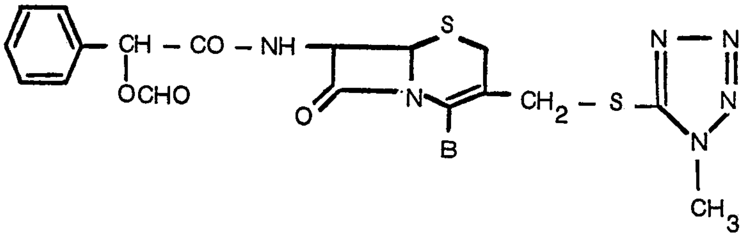

(IIc)

worin B ein Carbonsäureesterrest ist, den Esterrest der Verbindung (IIc) abspaltet und danach die Verbindung (IIc) mit einer alkalischen Lösung behandelt.

7. Verfahren nach den Ansprüchen 3 bis 6, worin man die Reaktion in Gegenwart eines Lösungsmittels durchführt.

8. Verfahren nach den Ansprüchen 3 bis 6, worin man das Salz der Verbindungen der Formeln (II) und (IIIa) aus der aus Alkalimetallen, Erdalkalimetallen und stickstoffhaltigen organischen Basen bestehenden Gruppe auswählt.

9. Verfahren nach Anspruch 8, worin man das Salz aus der aus Natrium-, Kalium-, Calcium-, Magnesium-, Trimethylamin-, Triethylamin-, Pyridin-, N-Methylpiperidin- und N-Methylmorpholinsalz bestehenden Gruppe auswählt.

10. Verfahren nach den Ansprüchen 3 bis 6, worin man die Reaktionen bei einem pH-Wert von 1 bis 10 durchführt.

11. Verfahren nach den Ansprüchen 3 bis 6, woin A ein Benzimidazol-2-ylthiorest ist.

12. Verfahren nach den Ansprüchen 3 bis 6, worin A ein 1-Methyl-1H-tetrazol-5-ylthiorest ist.

13. Verfahren nach den Ansprüchen 3 bis 6, worin man die Reaction der Verbindung (Ia) oder (Ib) und den Verbindung (IIIa) oder (IIIb) durchführt, indem man einen Katalysator aus einer anorganischen Säure oder einer organischen Säure verwendet.

14. Verfahren nach Anspruch 13, worin die anorganische Säure Salzsäure oder Schwefelsäure ist.

15. Verfahren nach Anspruch 13, worin die organische Säure 1-Methyl-1H-tetrazol-5-thiol ist.